# EUROPÄISCHE PATENTANMELDUNG

(11) **EP 1 192 918 A1**
(43) Veröffentlichungstag der Anmeldung: **03.04.2002**
(21) Anmeldenummer: 01111869.2
(22) Anmeldetag: 16.05.2001
(51) Int. Cl.: A61F 5/455

(54) **Harnableitvorrichtung für weibliche Personen**

(30) Priorität: 31.08.2000 DE 20015055 U
(71) Anmelder: Grundke, Reinhold, 84489 Burghausen (DE); Liedl, Bernhard, Dr. med., 81337 München (DE); Jaeniche, Wilhelm, 77694 Kehl-Leutesheim (DE)
(72) Erfinder: Grundke, Reinhold, 84489 Burghausen (DE); Liedl, Bernhard, Dr. med., 81337 München (DE); Jaeniche, Wilhelm, 77694 Kehl-Leutesheim (DE)
(74) Vertreter: Finck, Dieter, Dr.Ing.

(57) **Zusammenfassung**

Die Harnableitvorrichtung für weibliche Personen besteht aus einem länglichen Hohlkörper (10) aus flexiblem Material der eine Bodenfläche (11) mit einem, mit einem Schlauch verbindbaren Ablauf (12), zwei Längswände (13, 14) und zwei Stirnwände (15, 16), die mit der Bodenfläche (11) ein Stück bilden, und eine offene Oberseite mit einem um die Scheide herum zu positionierenden durchgehenden, im wesentlichen in einer Ebene liegenden oberen Rand (17) aufweist, von dem seitlich nach außen ein umlaufender flexibler Flansch (28) für eine schenkelseitige Abdichtung absteht. An der einen Stirnwand (15) ist bündig mit dem oberen Rand abschließend eine mittige, den Flansch (28) dort ersetzende und von ihm getrennte, flexible bandförmige Lasche (20) angeformt, die an ihrem freien Ende eine Befestigungseinrichtung (21) trägt und für ein schambeinseitiges Anliegen vorgesehen ist. An der anderen Stirnwand (16) ist den Flansch (28) dort ersetzend und von ihm getrennt mittig eine weitere flexible bandförmige Lasche (22) so angeformt, daß die mit einem ersten Abschnitt (24) für ein abdichtendes Anliegen am Damm (34) im wesentlichen senkrecht zum Rand (17) von der offenen Seite des Körpers weg vorsteht und in einen zweiten Abschnitt (25) übergeht, der für ein Liegen in der Gesäßfalte (32) vorgesehen ist und mit seinem freien Ende eine Befestigungseinichtung (23) trägt.

## Beschreibung

Die Erfindung betrifft eine Harnableitvorrichtung für weibliche Personen.

Die Harninkontinenz ist ein außerordentlich belastendes Symtom. Urin ist häufig alkalisch und zerstört den pH-Säuremantel der Haut. Je nach Menge des abgehenden Urins kann davon ein großflächiges Hautgebiet betroffen sein und je länger der Urin darauf verbleibt, desto größer ist die Gefahr von Hautirritationen.

Unkontrolliert abgehender Urin führt an der Wäsche, am Körper, aber auch im weiten Umfeld zu einer außerordentlichen, abstoßenden Geruchsbelästigung. Ständig mit Urin benetzte Haut kann mazerieren und unterschiedliche Dermatosen entwickeln bis hin zu Decubitalulcera. Eine ordentliche Inkontinenzversorgung ist daher von immenser Bedeutung.

Zur Verfügung stehen heute eine Reihe von Vorlagen unterschiedlicher Größe und Saugfähigkeit. Die Notwendigkeit häufigen Wechsels, das Aufkommen einer erheblichen Menge nach Urin stinkenden Mülls, die äußere Sichtbarkeit zumindest größerer Vorlagen und die Gefahr einer Haut-Mazeration und -infektion sind Nachteile dieses Verfahrens.

Hautmazerationen bis hin zur Ulkusbildung stellen bei dieser Form der Inkontinenzversorgung nach wie vor ein Problem dar, so daß in vielen Fällen ein Harnröhrendauerkatheterismus vorgezogen wird. Die Beherrschung der Harninkontinenz wird dann allerdings mit der zunehmenden Gefahr von Harnweginfektionen, Striktur- und Fistelbildungen und mit dem Fremdkörperreiz eines liegenden Harnröhrenkatheters erkauft. Kommen dann zu altersbedingten Veränderungen der Haut noch der inkontinenzbedingte häufige Kontakt mit Urin und vielleicht sogar Stuhl und die notwendigen Waschungen hinzu, ist die Haut stark strapaziert und gefährdet.

Besondere Probleme ergeben sich aufgrund der großen Hautfalten im Genitalbereich und aufgrund der Gesäßfalte. In diesen Arealen besteht dauernd oder zeitweise ein direkter Kontakt von Haut zu Haut in einem permanent feuchten Milieu, das Mazerationen begünstigt. In Verbindung mit einer Veränderung des Säuremantels der Hautoberfläche sind dies ideale Voraussetzungen für krankheitsauslösende Bakterien und Pilze, insbesondere für Candida albicans.

Die der Erfindung zugrundeliegende Aufgabe besteht deshalb darin, eine Harnableitvorrichtung für weibliche Personen bereitzustellen, die einfach zu handhaben und anzuwenden ist, eine Wäscheverschmutzung bei sachgemäßer Anwendung ausschließt, die Bewegungsfreiheit des Patienten so wenig wie möglich beeinträchtigt und zudem wiederholt verwendbar ist.

Diese Aufgabe wird erfindungsgemäß durch eine Harnableitvorrichtung für weibliche Personen mit einem länglichen hohlen Körper aus flexiblem Material gelöst, der eine Bodenfläche mit einem mit einem Schlauch verbindbaren Ablauf, zwei Längswände und zwei Stirnwände, die mit der Bodenfläche ein Stück bilden, und eine offene Oberseite mit einem um die Scheide herum zu positionierenden durchgehenden oberen Rand aufweist, von dem seitlich nach außen ein umlaufender flexibler Flansch für eine schenkelseitige Abdichtung absteht, wobei an der einen Stirnwand bündig mit dem oberen Rand abschließend eine mittige, den Flansch dort ersetzende und von ihm getrennte flexible bandförmige Lasche angeformt ist, die an ihrem freien Ende eine Befestigungseinrichtung trägt und für ein schambeinseitiges Anliegen vorgesehen ist, und wobei an der anderen Stirnwand den Flansch dort ersetzend und von ihm getrennt mittig eine weitere flexible bandförmige Lasche so angeformt ist, daß sie mit einem ersten Abschnitt für ein abdichtendes Anliegen am Damm im wesentlichen senkrecht zum Rand von der offenen Seite des Körpers weg vorsteht und in einem zweiten Abschnitt übergeht, der für ein Liegen in der Gesäßfalte vorgesehen ist und an seinem freien Ende eine Befestigungseinrichtung trägt.

In dem von dem oberen Rand seitlich nach außen abstehenden flexiblen Flansch können durchgehende Löcher ausgebildet sein. Diese Löcher reduzieren die Kontaktfläche mit der Haut, verbessern jedoch die Haftung auf der Haut und ermöglichen eine Belüftung der Leistenfalte, wenn sich der Flansch abdichtend an der Haut leistenbogenseitig anlegt.

Direkt unterhalb des Flansches und der Laschen kann an den Längswänden und Stirnwänden durchgehend ein Stützsteg angeformt sein, der für den Eingriff mit dem Umfangsrand einer entsprechend angepaßten Öffnung in einer Miederhose dient.

Der von dem oberen Rand seitlich abstehende Flansch kann durch sich von außen zum Rand im wesentlichen senkrechte Einschnitte in flexible benachbarte Flanschelemente unterteilt werden. Dadurch wird eine bessere Anpassung des aus den Flanschelementen bestehendes Flansches an der Haut der Trägerin an den Innenseiten der Schenkel unterhalb des Leistenbogens gewährleistet, wobei seitliche Überlappungen, die den Tragekomfort beeinträchtigen würden, im wesentlichen ausgeschlossen bleiben.

Der erste Abschnitt der dammseitigen Lasche besteht bevorzugt aus der Abschlußwand eines im Querschnitt dreiwandigen Tunnelprofils, dessen offene Seite beim Tragen der Vorrichtung von der Gesäßspalte abgewandt, d. h. dem Ablauf zugewandt ist. Durch ihre Erhöhung über den Rand des Körpers verhindert die Lasche eine Harnvernetzung im Dammbereich und schließt somit Hautirritationen in diesem Gebiet aus. Die Ausgestaltung der Lasche in Form des Tunnelprofils ergibt eine gute Einpassung in die Gesäßfalte, wobei eine ausreichende Elastizität dadurch gewährleistet ist, daß die Seitenwände des Tunnelprofils problemlos elastisch aufeinander zu und voneinander weg gebogen werden können.

Der Ablauf ist zweckmäßigerweise in der Bodenfläche nahe an der dammseitigen Stirnwand trichterförmig ausgebildet und geht in einen Ablaufrohrstutzen über. Wenn die Harnableitvorrichtung getragen wird, ist die Bodenfläche zum Ablauf hin geneigt. Dadurch ist eine vollständige Entleerung des Körpers möglich.

In der schambeinseitigen Stirnwand wird vorteilhafterweise eine der Be- und Entlüftung dienende Öffnung vorgesehen, die so weit unter dem oberen Rand angeordnet ist, daß sie nicht mit dem Schambein in Verschlußkontakt bringbar ist und über sie kein Harn ausfließen kann. Diese Be- und Entlüftung mittig unterhalb des Schambeins ermöglicht den Abzug von feuchter Luft, verringert dadurch die Mazerationsgefahr der Haut und begünstigt den Harnabfluß in das Ablaufrohr.

Der Körper mit seinem Flansch bzw. seinen Flanschelementen, seinen beiden Laschen und seinem Ablaufrohrstutzen besteht vorteilhafterweise aus Silikonkautschuk, also aus einem weichelastischen, im wesentlichen antibakteriellen, leicht reinigbaren Material.

Die Befestigungseinrichtungen an den freien Enden der Laschen bestehen zweckmäßigerweise aus Klettverschlüssen, die auf einfache Weise mit einem Hüfttragegürtel verbindbar sind, der sich im Bereich des dritten und vierten Lendenwirbels um den Krörper der Trägerin herum erstreckt.

Bevorzugt ist der längliche hohle Körper mit seiner offenen Oberseite badenwannenförmig ausgebildet, d. h. sein Boden und seine Stirnwände sind jeweils abgerundet, wobei sich die Querschnittsfläche des Körpers zum Ablaufrohr hin etwas verkleinert, indem die Längswände etwas konvergieren.

Die erfindungsgemäße Harnableitvorrichtung ist aufgrund ihrer Wiederverwendbarkeit umweltfreundlich. Sie sollte täglich in einer Waschmaschine bei 60 bis 90° gewaschen werden, wodurch Auflagerungen, wie Biofilme oder Inkrustierungen vermieden werden sollen. Makroskopische Auflagerungen können mechanisch entfernt werden. Eine Dampfsterilisation ist nicht oder nur selten erforderlich.

Vor dem Anlegen der Harnableitvorrichtung wird der Hüfttragegürtel mit der Befestigungseinrichtung der dammseitigen Lasche über den Klettverschluß verbunden. Dann wird der Hüfttragegürtel angelegt und anschließend die Harnableitvorrichtung zwischen den Schenkel hindurch zum Schambein geführt und die schambeinseitige Lasche mit ihrer Befestigungseinrichtung in Form des Klettverschlusses an dem Hüfttragegürtel festgelegt. Durch die Badewannenform ist der Rand der offenen Oberseite des Körpers so abgestimmt, daß er die Scheide umschließt, wobei sich die Flanschelemente im Leistenbogen elastisch abbiegen und abdichtend an die Haut der Schenkel anliegen, wobei schambeinseitig durch die zugeordnete Lasche, dammseitig durch den ersten, hochstehenden Abschnitt und gesäßseitig durch den zweiten Abschnitt mit der tunnelförmigen Ausgestaltung die erforderliche Abdichtung erreicht wird. Zur Stützung der Positionierung des so angelegten Körpers kann die Trägerin noch eine Miederhose anziehen, die mit dem Rand der im Schritt vorgesehenen Aussparung an dem Stützsteg anliegt und dadurch die Halterung und Fixierung nach oben verbessert. Es braucht dann nur noch die Schlauchverbindung zwischen dem Ablaufrohrstutzen und einem Beinbeutel hergestellt zu werden. Die Harnableitvorrichtung kann nicht nur im Liegen sondern auch im Stehen, beim Gehen und im Sitzen getragen werden.

Anhand von Zeichnungen wird eine beispielsweise Ausführungsform der Erfindung näher erläutert. Es zeigt:
- Fig. 1: eine erfindungsgemäße Harnableitvorrichtung perspektivisch von unten,
- Fig. 2: eine Draufsicht auf die Harnableitvorrichtung von Fig. 1,
- Fig. 3: eine Seitenansicht der Harnableitvorrichtung von Fig. 1 und 2,
- Fig. 4: den Schnitt A-A von Fig. 1 bzw. 2 und
- Fig. 5: die von dem Kreis B eingeschlossene Einzelheit von Fig. 3.

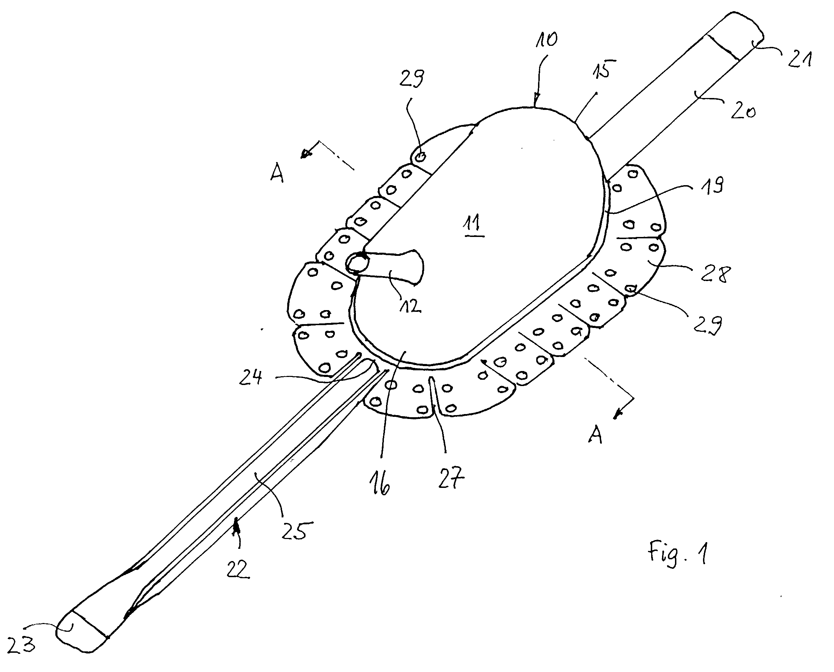

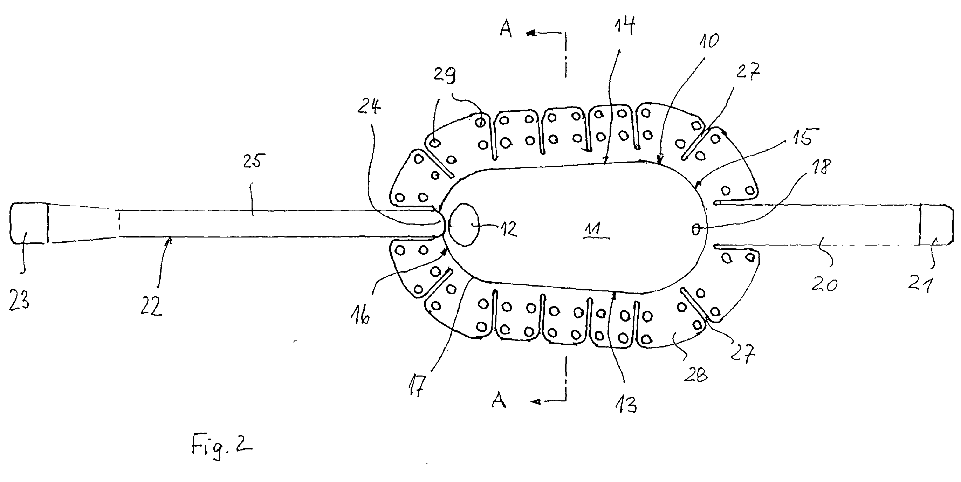

Die in Fig. 1 bis 5 gezeigte Harnableitvorrichtung hat einen länglichen hohlen Körper 10, der im wesentlichen die stark verkleinerte Form einer Badewanne hat. Der Körper 10 hat eine abgerundete Bodenfläche 11 mit einer etwas größeren abgerundeten Stirnwand 15 und einer gegenüberliegenden etwas kleineren abgerundeten Stirnwand 16, die durch Längswände 13 und 14 verbunden sind, die abgerundet in die Bodenfläche 11 übergehen. Die Längswände 13 und 14 verlaufen dadurch leicht konvergierend zur kleineren Stirnwand 16 hin. An dem Übergang der Bodenfläche 11 in die kleinere Stirnwand 16 ist als Ablauf eine trichterförmige Öffnung vorgesehen, die in einen Ablaufrohrstutzen 12 mündet.

Die beiden Stirnwände 15 und 16 und die beiden Längswände 13 und 14 haben einen in einer Ebene liegenden oberen Rand 17, an dem ein sich seitlich nach außen erstreckender Flansch aus dem gleichen Material wie der Körper 10 angeformt ist. Der Flansch ist durch im wesentlichen senkrecht zum oberen Rand 17 hin gerichtete Einschnitte in voneinander beabstandete Flanschelemente 28 unterteilt, die sich um den gesamten Umfang des oberen Flanschs 17 herum erstrecken, ausgenommen die beiden Mitten der Stirnwände 15, 16, an denen Laschen 20 bzw. 22 aus dem gleichen Material angeformt und ebenfalls durch Einschnitte 27 von den benachbarten Flanschelementen 28 getrennt sind. Jedes der Flanschelemente 28 ist mit durchgehenden Löchern 29 versehen.

Unterhalb des oberen Randes 17 erstreckt sich auf der Außenseite der Längswände 13 und 14 und der Stirnwände 15 und 16 ein durchgehender Stützsteg 19, mit welchem der Rand einer entsprechenden, in einer Miederhose ausgesparten Öffnung nach dem Anlegen der Harnableitvorrichtung in Eingriff bringbar ist, wobei die Miederhose nicht gezeigt ist.

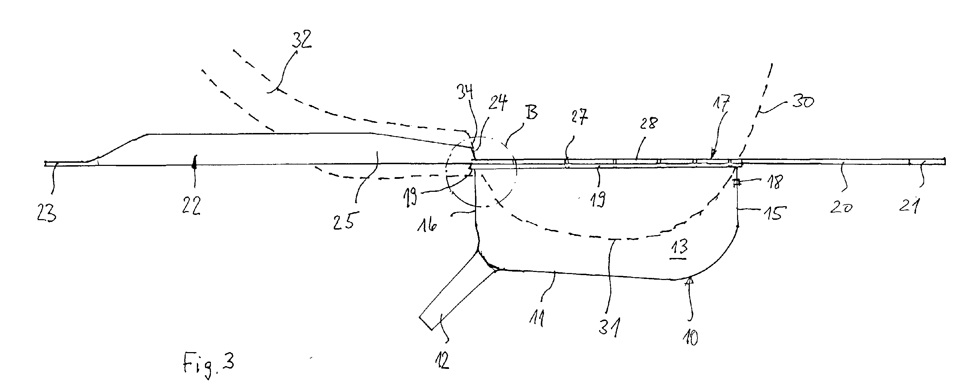

Die bündig mit dem oberen Rand 17 mittig von der größeren Stirnwand 15 abstehende Lasche 20 ist bandförmig und flexibel ausgebildet und hat an ihrem freien Ende eine Befestigungseinrichtung 21 in Form eines Klettverschlusses. Die bandförmige Lasche 20 ist für ein Anliegen auf dem Schambein 30 (Fig. 3) vorgesehen.

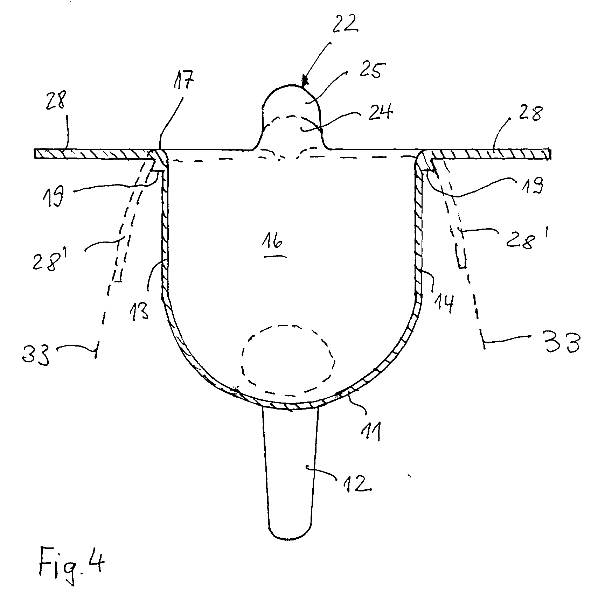

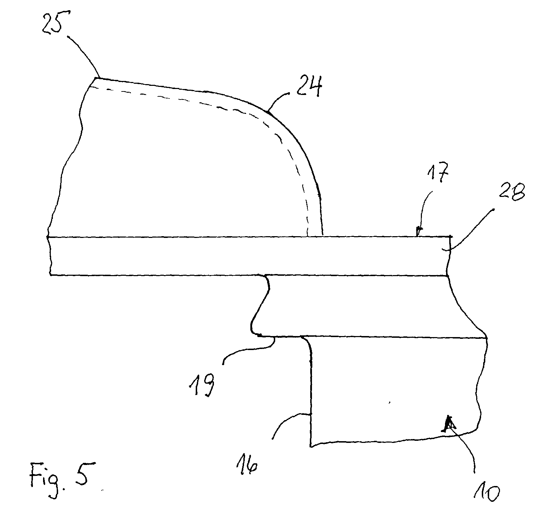

Die von der kleineren Stirnwand 16 ausgehende Lasche 22 hat einen, wie aus Fig. 4 und 5 zu sehen ist, nach oben abstehenden ersten Abschnitt 24, der in einen Abschnitt 25 übergeht, der ein dreiwandiges tunnelförmiges Profil aufweist, dessen offene Seite dem Ablaufrohrstutzen 12 zugewandt ist. Der erste Abschnitt 24 bildet dabei die körperseitige Abschlußwand des Tunnelprofils des zweiten Abschnitts 25. Die Lasche 22 hat an ihrem freien Ende eine von einem Klettverschluß gebildete Befestigungseinrichtung 23. Wie aus Fig. 3 zu sehen ist, dient der Abschnitt 24 der Lasche 22 zum abdichtenden Anliegen an dem Damm 34 der Trägerin, während der zweite Abschnitt 25 mit seinem tunnelförmigen Profil von der Gesäßfalte 32 aufgenommen wird.

Die in Fig. 3 gestrichelt eingezeichnete Vulva 31 befindet sich innerhalb des Körpers 10, wobei dessen Bodenfläche 11, wenn der Körper mit seinen Laschen 20 und 22 an der Trägerin positioniert ist, leicht zum Ablaufrohr 12 geneigt ist, das sich dann an der tiefsten Stelle befindet.

An der größeren Stirnwand 15, von der aus sich die schambeinseitige bandförmige Lasche 20 erstreckt, ist etwas unterhalb des Stützstegs 19 mittig eine Öffnung 18 für ein Belüften und Entlüften des Innenraums des Körpers 10 ausgebildet. Der Abstand der Öffnung 18 zum Stützsteg ist so bemessen, daß, wenn die Harnableitvorrichtung von der Trägerin getragen wird, die Öffnung 18 noch keinen Kontakt mit dem Schambein 30 hat, so daß die Funktion der Öffnung 18 gewährleistet bleibt, jedoch wegen ihrer Obenlage kein Harn durch sie ausfließen kann.

Wenn die Harnableitvorrichtung angelegt wird, wird zunächst an einem nicht gezeigten Hüfttrageband die Befestigungseinrichtung 23 in Form eines Klettverschlusses festgelegt, dann das Hüfttrageband etwa in Höhe des 3. bis 4. Lendenwirbels angelegt und die Harnableitvorrichtung zwischen den Beinen der Trägerin gehalten an der schambeinseitigen bandförmigen Lasche 20 hindurchgeführt und mit der Befestigungseinrichtung 21 dieser Lasche 20 in Form eines Klettverschlusses am Hüfttragegürtel festgelegt.

Wenn die Harnableitvorrichtung in dieser Weise von der Trägerin angelegt worden ist, nehmen die Flanschelemente 28 die in Fig. 4 gestrichelt gezeichnete Position 28' ein und liegen dabei abdichtend im wesentlichen aneinander und anliegend an der Haut 33 an. Der hochstehende erste Abschnitt 24 der tunnelförmigen dammseitigen Lasche 22 liegt dichtend zwischen den benachbarten Flanschelementen 28 an dem Damm 34 an, während sich der zweite Abschnitt mit dem Tunnelprofil innerhalb der Gesäßspalte 32 erstreckt und aufgrund seiner Flexibilität seine Form in Richtung der Breite der Gesäßspalte anpassen kann. Die bandförmige Lasche 20 liegt an dem Schambein 30 an, während sich die Vulva 31 mit dem Harnauslaß innerhalb des Hohlraums des Körpers 10 befindet, der randseitig überall abdichtend am Körper anliegt, so daß in den Hohlraum des Körpers eintretender Harn über den Ablaufrohrstutzen 12 und einen nicht gezeigten Schlauch in einen Sammelbehälter abfließen kann, der beispielsweise um ein Bein geschnallt getragen wird.

Die Löcher 29 in den Flanschelementen 28 verringern einerseits die Kontaktfläche mit der Haut, gewährleisten andererseits eine Belüftung und erhöhen die Fixierung der Flanschelemente 28 an der Haut dadurch, daß sich die Haut etwas in die Löcher 28 hineindrückt.

Die Abmessungen der Harnableitvorrichtung sind an die Durchschnittsabmessungen der entsprechenden Körperpartien der Trägerin angepaßt. Es können Übergrößen oder kleinere Größen hergestellt werden. Die Befestigungseinrichtungen 21 und 23 lassen sich in ihrer Länge einfach anpassen.

Die Harnableitvorrichtung läßt sich mit Ausnahme der Klettverschlüsse in einem Stück aus Silikonkautschuk ausformen. Silikonkautschuk ist ein sehr flexibles und elastisches Material, das mit dem Körper gut verträglich ist und sich unter dem Einfluß der Körperflüssigkeit nicht zersetzt und mit heißem Wasser leicht und vollständig gereinigt werden kann.

## Patentansprüche

1. Harnableitvorrichtung für weibliche Personen mit einem länglichen Hohlkörper (10) aus flexiblem Material
- der eine Bodenfläche (11) mit einem ,mit einem Schlauch verbindbaren Ablauf (12),
- zwei Längswände (13, 14) und zwei Stirnwände (15, 16), die mit der Bodenfläche (11) ein Stück bilden,
- eine offene Oberseite mit einem um die Scheide herum zu positionierenden durchgehenden, im wesentlichen in einer Ebene liegenden oberen Rand (17) aufweist, von dem seitlich nach außen ein umlaufender flexibler Flansch (28) für eine schenkelseitige Abdichtung absteht,
- wobei an der einen Stirnwand (15) bündig mit dem oberen Rand abschließend eine mittige, den Flansch (28) dort ersetzende und von ihm getrennte, flexible bandförmige Lasche (20) angeformt ist, die an ihrem freien Ende eine Befestigungseinrichtung (21) trägt und für ein schambeinseitiges Anliegen vorgesehen ist, und
- wobei an der anderen Stirnwand (16) den Flansch (28) dort ersetzend und von ihm getrennt mittig eine weitere flexible bandförmige Lasche (22) so angeformt ist, daß die mit einem ersten Abschnitt (24) für ein abdichtendes Anliegen am Damm (34) im wesentlichen senkrecht zum Rand (17) von der offenen Seite des Körpers weg vorsteht und in einen zweiten Abschnitt (25) übergeht, der für ein Liegen in der Gesäßfalte (32) vorgesehen ist und mit seinem freien Ende eine Befestigungseinichtung (23) trägt.

2. Harnableitvorrichtung nach Anspruch 1, **gekennzeichnet durch** in dem von dem oberen Rand (17) seitlich nach außen abstehenden flexiblen Flansch (28) ausgebildete durchgehende Löcher (29).

3. Harnableitvorrichtung nach Anspruch 1 oder 2, **gekennzeichnet durch** einen direkt unterhalb des Flansches (28) und der Laschen (20, 22) durchgehend an den Längswänden (13, 14) und Stirnwänden (15, 16) angeformten Stützsteg (19) für den Eingriff mit dem Umfangsrand einer entsprechend ausgesparten Öffnung in einer Miederhose.

4. Harnableitvorrichtung nach einem der vorhergehenden Ansprüche, **dadurch gekennzeichnet, daß** der von dem oberen Rand (17) seitlich abstehende Flansch durch sich von außen zum Rand (17) im wesentlichen senkrecht erstreckende Einschnitte (27) in flexible benachbarte Flanschelemente (28) unterteilt ist.

5. Harnableitvorrichtung nach einem der vorhergehenden Ansprüche, **dadurch gekennzeichnet, daß** der erste Abschnitt (24) der dammseitigen Lasche (22) aus der Abschlußwand eines im Querschnitt dreiwandigen Tunnelprofils besteht, dessen offene Seite dem Ablauf zugewandt ist.

6. Harnableitvorrichtung nach einem der vorhergehenden Ansprüche, **dadurch gekennzeichnet, daß** der Ablauf (12) in der Bodenfläche (11) nahe an der dammseitigen Stirnwand (16) trichterförmig ausgebildet ist und in einen Ablaufrohrstutzen übergeht.

7. Harnableitvorrichtung nach einem der vorhergehenden Ansprüche, **gekennzeichnet durch** eine in der schambeinseitigen Stirnwand (15) vorgesehene, der Be- und Entlüftung dienende Öffnung (18), die so weit unter dem oberen Rand (17) angeordnet ist, daß sie nicht mit dem Schambein (30) in Verschlußkontakt bringbar ist und über sie kein Harn ausfließen kann.

8. Harnableitvorrichtung nach einem der vorhergehenden Ansprüche, **dadurch gekennzeichnet, daß** der Körper (10) mit seinem Flansch bzw. seinen Flanschelementen (28), seinen Laschen (20, 22) und seinem Ablauf (12) in einem Stück aus Silikonkautschuk ausgeformt ist.

9. Harnableitvorrichtung nach einem der vorhergehenden Ansprüche, **dadurch gekennzeichnet, daß** die Befestigungseinrichtungen (21, 23) mit einem Hüfttragegürtel verbindbar sind.

10. Harnableitvorrichtung nach Anspruch 9, **dadurch gekennzeichnet, daß** die Befestigungseinrichtungen (21, 23) Klettverschlüsse sind.

11. Harnableitvorrichtung nach einem der vorhergehenden Ansprüche, **dadurch gekennzeichnet, daß** der längliche hohle Körper mit seiner offenen Oberseite die Form einer miniaturisierten Badewanne hat.
